# EUROPEAN PATENT APPLICATION

(11) **EP 2 797 056 A1**
(43) Date of publication of application: **29.10.2014**
(21) Application number: 13165264.6
(22) Date of filing: 25.04.2013
(51) Int. Cl.: G07F 17/32, A61B 5/00, G06F 19/00, A63K 3/00, A01K 11/00, A01K 15/02

(54) **Arrangement for processing data of sport animals**

(71) Applicant: TimerGPS Europe Oy, 90400 Oulu (FI)
(72) Inventor: Heiskanen, Kalle, 90400 Oulu (FI); Heiskanen, Ville, 90400 Oulu (FI)
(74) Representative: Määttä, Jukka Tapani

(57) **Abstract**

The invention relates to an arrangement for processing, analysing, collecting, and utilizing sport animal training related data. The invention also relates to a method for processing, analysing, collecting and utilizing sport animal training related data. The invention further relates also to a server for processing, collecting, analysing, and combining sport animal training related data from sources that are utilized in the configuration of a sport animal management device, which can be used flexibly to multiple animals with multiple trainers on the basis of configurability achieved with the server.

## Description

### Technical Field

The invention relates to an arrangement for processing, analysing, collecting, and utilizing sport animal training related data. The invention also relates to a method for processing, analysing, collecting and utilizing sport animal training related data. The invention further relates also to a server processing, analysing collecting, and combining sport animal training related data from sources and to a sport animal management device configured based on the analysed data.

### Background

There is a huge amount of data related to different types of sport animals such as dogs, horses for different types of sports, reindeer, camels, or oxen, for example. The data is produced during training events, daily care, different competitions, veterinarian checks and animal breeding, for example. The problem related to the data is that it is very rarely collected from different sources, combined and stored in a centralized manner anywhere. Instead, the data is spread widely around different types of computer files, databases, digital and non-digital notebooks, other media apparatuses and information systems.

For one single sport animal there could be dozens of different sources in which the data of the animal is stored. Obtaining an overview or, furthermore, creating a fact based training program and presenting the current status of the sport animal to its owners or trainer becomes complicated. There are also different types of training devices that can used to assist and monitor different training events. The problem with the data leads to situations in which training devices as such are not configured according to fully analysed and combined data for the specific sport animal and therefore the devices either cannot suggest an analysed training program or can only partially suggest a program according to the data stored in the specific training device. Accuracy of the data used for the training program planning is important and it suffers if the same training device is used by several sport animals and the device cannot be configured for each animal specifically for their training events.

Also, sport animals such as different types of racehorses, for example, often have several owners. The problem with decentralized and widely spread data of the sport animals becomes evident as the owners cannot follow up the development and health of their animals easily online and give their feedback or instructions to trainers of their sport animals. Another problem with the widely spread and decentralized data is that comparative data becomes difficult to obtain and utilize. For example, a simple comparison of training related information to competition results and obtaining reference data from other sport animals with similar level of training, age, or gender with statistical accuracy is currently not provided comprehensively. Also, a single sport animal can have more than one trainer and the data related to training events with different trainers is not easily available and centralized to all the trainers which causes lack of knowledge during different training events.

The earlier solutions have not provided a complete solution to solve the problem described above. Publication US2006173367 (A1) describes an apparatus for determining the health or fitness, under an exercise load, of an animal (such as a horse) and suggests that lameness, disease or poor physiological potential of the animal can be identified with the apparatus. Another publication, US7062895 (B1), presents an electronic programmable timing instrument for real-time monitoring of an equine athlete's training. Conditioning regimen for developing optimum racing potential is provided wherein the instruments aid an operator primarily to set, monitor and control the equine athlete's distance and speed. Secondarily, the presented apparatus is suggested to monitor and evaluate physiological parameters that are used to assess physical stress of the animal in real time and include security parameters for protecting proprietary collected data. The solution is suggested to make possible a new method for training equine athletes and for tracking their development and potential problems therewith.

Both of the above presented publications are only partial solutions for monitoring the status of a sport animal and training them effectively. These solutions aim to offer a device for tracking and recording certain parameters during training. These solutions are not connected into a wider arrangement that would allow combining training, competition and health related data from different sources and providing a complete status analysis for a sport animal and suggesting, for example, a training program based on that analysis in the device. These solutions lack the possibility to be configured based on a more widely combined data for a sport animal.

### Summary of the Invention

The object of the present invention is to offer a solution in which sport animal data from different data sources, such as sport animal management devices, different digital databases and different media apparatuses, is combined and statistically analysed in order to provide analysed information on a sport animal describing a complete status on the training and health of the animal, management and care of the animal, such as feeding, shoeing or required check-ups with a veterinarian, like vaccinations, suggesting a training program and instructing a trainer of the animal. Another object of the present invention is to provide instant configurability of a sport animal management device for a training event of a certain sport animal based on utilizing the analysed information about the sport animal and through configurability enable the sport animal management device to instruct the trainer of the animal of the training program. Also, the object of the present invention is to enable presentation of the analysed information for a certain sport animal in the sport animal management device and to enable a mechanism for providing feedback related to the sport animal through the device. Further, the object of the present invention is to allow use of one sport animal management device to more than one animal effectively through utilizing the instant configurability.

The objects of the present invention are fulfilled by providing an arrangement for processing, collecting, analysing and utilizing sport animal training related data, the arrangement comprising:
at least one server configured to receive data related to at least one sport animal from at least one sport animal training device or at least one media apparatus or at least one digital database and process and analyse the received data related to at least one sport animal;
at least one sport animal management device configured to send and receive information request or management information for configuration of the device related to at least one sport animal to and from at least one server, display the information received from the server, record training event data or management information related to at least one sport animal and send the recorded data to at least one server; and
at least one media apparatus or at least one digital database for providing at least competition data, veterinarian data and service provider data related to at least one sport animal;
characterised in that the server further comprises means for combining and statistically analysing the sport animal related data received from at least one sport animal management device, at least one media apparatus or at least one digital database in order to provide analysed sport-animal-specific information in the server and configuring a sport animal management device based on the analysed sport-animal-specific information received from the server.

Further, the objects of the present invention are fulfilled by providing a server for receiving, analysing, collecting, processing and further sending sport animal training related data that is utilized in configuration of a sport animal management device, the server comprising:
a data communication interface;
one or more processors;
one or more memories including computer program code;
the one or more memories and the computer program code configured to, with the one or more processors, cause the server at least to receive with the data communication interface from at least one sport animal management device data related to at least one training event of a sport animal, receive with the data communication interface from at least one media apparatus data related to at least one sport animal or receive with the data communication interface from at least one sport animal management device an information request for configuration of the said device related to at least one sport animal;
characterised in that the server further comprises means for sending with the data communication interface the analysed information of at least one sport animal to at least one sport animal management device in order to configure the sport animal management device.

Also, the objects of the present invention are fulfilled by providing a sport animal management device comprising:
a data communication interface;
one or more processors;
one or more memories including computer program code;
the one or more memories and the computer program code configured to, with the one or more processors, cause the sport animal management device at least to receive with the data communication interface from at least one server information related to at least one sport animal, send with the data communication interface an information request or management information related to at least one sport animal to at least one server and display the sport animal related information received from the server;
characterised in that the sport animal management device further comprises means for instructing a trainer of the sport animal about a training program or management information for the requested sport animal based on the analysed information received from the server.

In addition to the foregoing, the objects of the present invention are fulfilled by providing a method for processing, collecting, analysing and utilizing sport animal training related data, the method comprising:
receiving from at least one sport animal management device data related to at least one training event or management information of a sport animal in the server;
receiving from at least one media apparatus or digital database data related to at least one sport animal in the server;
sending an information request related to at least one sport animal from at least one sport animal management device to at least one server and receiving a response to the request in the sport animal management device; and
receiving from at least one sport animal management device an information request related to at least one sport animal in the server and responding to the request in the server;
characterised in that the method further comprises combining and statistically analysing the sport animal related data received from at least one sport animal management device, at least one media apparatus or at least one digital database in the server in order to provide analysed sport-animal-specific information and configuring a sport animal management device based on the analysed sport-animal-specific information received from the server.

Further scope of applicability of the present invention will become apparent from the detailed description given hereafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and accompanying drawings which are given by way of illustration only, and thus are not limitative of the present invention and wherein
- Fig. 1: shows a schematical representation of an arrangement for processing, collecting, analysing and utilizing sport animal training related data;
- Fig. 2: shows a flow chart describing an exemplary, high-level scenario for recording, processing, analysing, and combining training event related data or management information for a sport animal and utilizing information based on all the collected data in configuration of a sport animal management device;
- Fig. 3: shows a flow chart describing an exemplary, high-level scenario for providing management information for a sport animal, processing and combining the given information with other sport animal data and utilizing information based on all the collected data in configuration of a sport animal management device;
- Fig. 4: shows a flow chart describing exemplary, high-level method steps for a method of processing, collecting, analysing, and utilizing sport animal training related data;
- Fig. 5a: shows a use case based example of applying the arrangement in horse training and development follow-up;
- Fig. 5b: complements Figure 5a and presents the arrangement of data in case of the exemplary use case in Figure 5a.

### Detailed Description of the Drawings and Preferred Embodiments of the Invention

In the following description, considered embodiments are merely exemplary, and one skilled in the art may find other ways to implement the invention. Although the specification may refer to "an", "one" or "some" embodiment(s) in several locations, this does not necessarily mean that each such reference is made to the same embodiment(s), or that the feature only applies to a single embodiment or all embodiments. Single feature of different embodiments may also be combined to provide other embodiments.

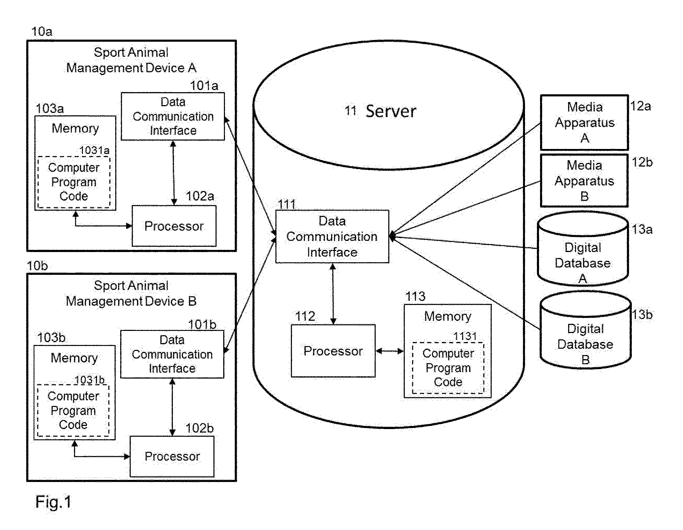

Figure 1 illustrates one advantageous embodiment of the invention presenting an exemplary arrangement for processing, collecting, analysing and utilizing sport animal training related data through essential components of the arrangement. The arrangement in this advantageous embodiment consists of a server 11, two sport animal management devices 10a, 10b, two media apparatuses 12a, 12b and two digital databases 13a, 13 b. The server 11 essentially comprises a data communication interface 111 enabling communication with sport animal management devices 10a, 10b through their data communication interfaces 101a, 101 b, a processor 112 and a memory unit 113 containing computer program code 1131. The server 11 can advantageously be implemented as a web server. The memory unit 113 and the computer program code 1131 together with the processor 112 cause the server 11 to receive through the data communication interface 111 from one of the sport animal management devices, 10a or 10b, data related to at least one training event of a sport animal or to receive with the data communication interface 111 from one media apparatuses, 12a or 12b, or digital databases, 13a or 13b, data related to at least one sport animal. The server 11 further comprises means for sending with the data communication interface 111 analysed information of at least one sport animal to one of the sport animal management devices, 10a or 10b, in order to configure that sport animal management device, 10a or 10b, for a training event for a certain sport animal. The server 11 may contain more than one processor 112 and there is no limitation on the number of sport animal management devices, 10a or 10b, media apparatuses, 12a or 12b, and digital databases, 13a or 13b, that can operate with the server 11. A sport animal management device, 10a or 10b, essentially comprises a data communication interface 101 a ,101 b enabling communication of the sport animal management devices, 10a or 10b, with the server 11, a processor 102a, 102b, and a memory unit 103a, 103b containing computer program code 1031 a, 1031 b. The memory unit 103a, 103b and the computer program code 1031 a, 1031 b together with the processor 102a, 102b enable the sport animal management device 10a, 10b to receive with the data communication interface 101 a, 101 b from at least one server 11 information related to at least one sport animal, send with the data communication interface 101 a, 101 b an information request or management information related to at least one specific sport animal to the server 11 and display the sport animal related information received from the server 11. The sport animal management device, 10a or 10b, further comprises means for instructing a trainer of the sport animal on a training program or management information for a certain sport animal based on analysed information received from the server 11. Also, the sport animal management device, 10a or 10b, is typically not used by one specific sport animal only. Instead, one sport animal management device is typically used by at least 1-4 sport animals but the number of sport animals using one device can vary and is not limited to any certain number of sport animals and for a certain animal different sport animal management devices can be used during different events. Advantageously, the sport animal management device is, for example, a smart phone or tablet device with an application that turns the smart phone or tablet into a sport animal management device, a specific recording and measurement device or another suitable device that can be adapted to function within the arrangement described.

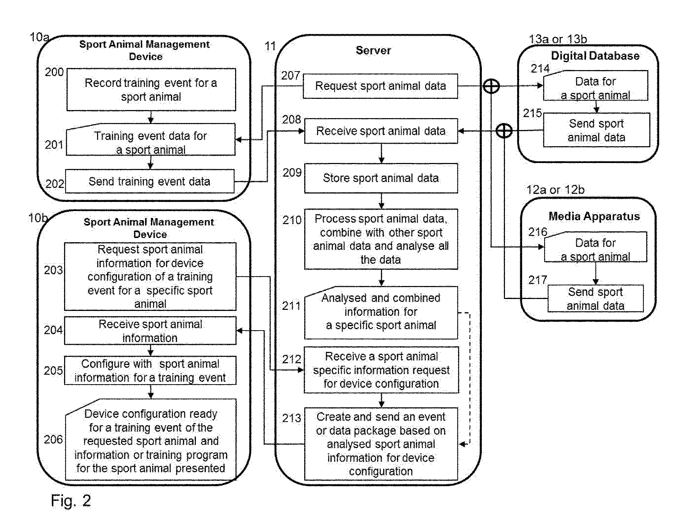

In relation to Figure 1 and also to Figures 2-4 it is essential that the sport-animal-specific information sent by the server 11 is advantageously used for configuring the sport animal management device 10a or 10b for a training event or displaying information of a certain sport animal, for example. Identification of a certain sport animal is achieved through a unique animal id that pairs all the data related to one sport animal with that specific sport animal and identifies the animal in all the requests, data recording and information sharing that take place either with the sport animal management device 10a, 10 b or the server 11. Also other means of identification for a certain sport animal when connecting data from different sources, such as 12a, 12b, 13a, or 13b, for example, to that specific animal in the server 11 can be used, such as at least date of birth, microchip id of the sport animal, and name of the sport animal. In addition to previous identification means, the data from different sources such as 10a, 10b, 12a, 12b, 13a, or 13b, for example, can also contain, for example, gender, competition data, veterinarian data, service provider data, family tree, feeding data, shoeing data, trainers, stable keeper, training programs, training done, training data, training index, participation of a sport animal in a certain competition, vaccination data of a sport animal, medical care of a sport animal, health condition of a sport animal, winnings of a sport animal, or results of the competitions of a sport animal. All the data related to a sport animal will be stored in the server. Sport animals in these examples can be different types of racehorses and also dogs, reindeer, camels, or oxen, for example but not limited to these.

Figure 2 illustrates one advantageous embodiment of the present invention through an exemplary data flow presentation for recording, processing, collecting, analysing and combining training event related data or management information for a sport animal and utilizing information based on all the collected data in the configuration of a sport animal management device. Training event for a sport animal is recorded in a sport animal management device 10a in step 200 and the data for the recorded training event is stored and available in the sport animal management device 201. A server 11 is responsible for collecting and managing all the sport animal related data for one or more sport animals from different data sources and apparatuses such as sport animal management devices 10a, different media apparatuses, 12a or 12b, or different digital databases 13a or 13b, for example. The number of data sources from which the data for sport animals is collected is varying and not limited to any certain number of sources. Advantageously, the server 11 requests sport animal related data, in step 207, from different data sources and devices such as 10a, 12a, 12b, 13a, or 13b, for example, that have suitable data 201, 214, 216 available. The server 11 can also receive the data automatically without a specific request based on the availability of the suitable data. The data sources and devices send back the training event data 202 and other sport animal related data 215, 217 that they hold. The server 11 receives the data sent to it 208, stores the data 209 based on the sport animal specific id or other identification data for sport animal as described previously above, such as date of birth, microchip id or name, stable name or nickname of the sport animal, for example. The purpose of storing the received data with identification means is to map the data concerning a certain sport animal that has been received from different data sources and devices 10a, 12a, 12b, 13a, or 13b with the data that already exists in the server concerning that specific sport animal. In step 210 the server 11 processes the received sport animal related data and combines it with other sport animal data that already exists in the server in order to create and maintain reference data and enable statistical analysis to be run on the data of a specific sport animal against the reference data available. Combining the received data with the existing reference data in the server and running statistical analysis provides analysed information on the training status and health of a specific sport animal as well as comparative information against the reference data for the specific sport animal as illustrated in step 211.

Further in the example of Figure 2 a request for sport animal information for device configuration of a training event of a specific sport animal is initiated, in step 203, from a sport animal management device 10b. The request advantageously contains a sport animal id or another means of identification for the specific sport animal in order to identify the animal in question. When the server 11 receives the request, in step 212, it advantageously identifies the information available on the server that relates to the specific sport animal, in step 211, based on the animal id or another means for identification of the animal. The server 11 then creates an event or a specific data package for configuring the requesting sport animal management device based on the analysed information and sends the event or package to the requesting sport animal management device 213. After receiving the information sent by the server 11 in step 204, the sport animal management device 10b advantageously configures itself based on the received event or package for the requested sport animal in step 205. Optionally, the server 11 can check that the requestor has rights to receive the requested data due to some of the data potentially being secret or confidential. After the configuration the device is ready for the training event of the specific sport animal and displays the necessary information about the animal 206. The displayed information may contain information on the sport animal's health, previous training results, and a training program for the sport animal including possible comparison to the reference data from the server, for example. Based on the information the sport animal management device is also advantageously capable of suggesting changes to the previous training program and instructing the trainer of the sport animal concerning possible training options. The trainer or another user of the device can also change the suggested training program or instructions and accept or decline suggestions and instructions.

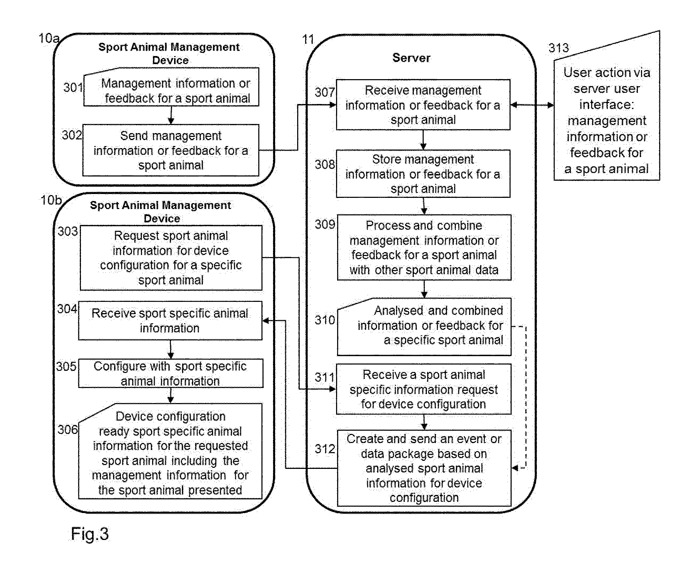

Figure 3 illustrates another advantageous embodiment of the present invention through an exemplary data flow presentation for providing management information or feedback for a sport animal, processing and combining the given information with other sport animal data and utilizing information based on all the collected data in the configuration of a sport animal management device. Management information for a specific sport animal can be provided and used by an owner, a trainer of the sport animal or another user of the system such as the service provider either through a web user interface 313 for the server or advantageously via a sport animal management device 301. The web user interface is advantageously available through smart phones, tablet devices, laptops, other portable devices with data communication capability, as well as computers. The web user interface also allows easy browsing and quick utilization of the information for various other purposes such as training program planning and adding data from other data sources, especially non-categorized or non-grouped free text type of data. The management information contains essentially information such as training instructions, notes and instructions for feeding, observations during competitions or training events or reminders on shoeing or health matters, estimations and estimation charts of performance of a specific sport animal related to training and competition statistics, and care and service needs and requests, for example, as well as identification means for the sport animal in question. Also, potential service providers can share information related to their services' availability through web user interface allowing wider access to the system. After the server 11 receives the given management information 307, it stores the information in step 308 based on data that identifies the specific sport animal such as a sport animal id. The server 11 processes the received management information and combines it with other sport animal data that already exists in the server 309. Combining the received management information with the existing reference data in the server advantageously provides a more comprehensive and analysed information over the status of the specific sport animal and enables feedback mechanism for training events 310, for example. Some of the information can advantageously be related to a group of animals such as horses with a certain stable keeper, dogs or horses of a certain race, or sport animals in certain geographical area, for example.

Further in the example of Figure 3 a request for sport animal information for device configuration of a specific sport animal according to step 303 for a training event, for example, is initiated from a sport animal management device 10b. The request advantageously contains a sport animal id or another means for identification for the specific sport animal or a group of animals such as date of birth, microchip id or name, stable name or nickname of the sport animal, for example, in order to identify the animal or group in question. When the server 11 receives the request in step 311, it advantageously identifies the information available on the server that relates to the specific sport animal, in step 310, based on the animal id and sends the analysed information together with the previously given management information related to that sport animal to the requesting sport animal management device 10b in a format of an event or a configuration package 312. After receiving the information, step 304, sent by the server 11 in a suitable format, the sport animal management device 10b advantageously configures itself based on the received information for the requested sport animal 305. After the configuration the device is ready for the specific sport animal and displays the information about the animal including also the given management information 306. The displayed information may contain information about the sport animal's health, previous training results, and a training program for the sport animal including possible comparison to the reference data from the server as well as any feedback, notes or instructions given by the owner or the trainer of the animal, for example.

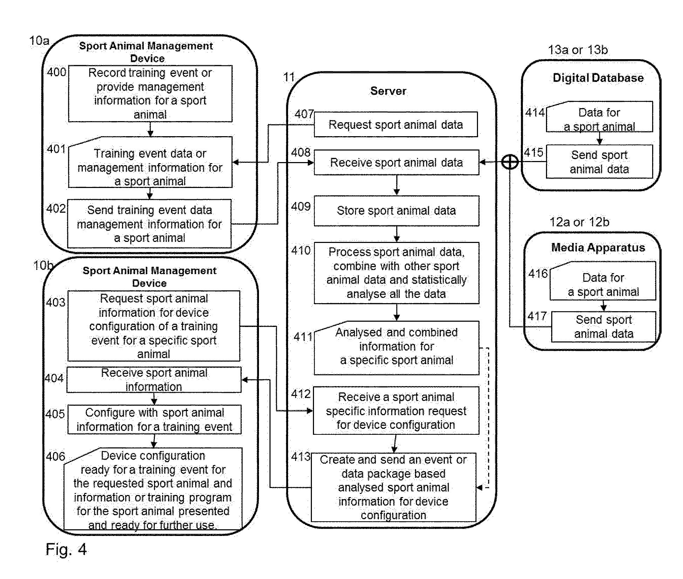

Further, Figure 4 illustrates an advantageous embodiment of the present invention through an exemplary data flow presentation for method steps of a method for processing and analysing sport animal training related data and utilizing the data in the configuration of a sport animal management device. Training event or management information for a sport animal is recorded in step 400 in a sport animal management device 10a. The recorded data or management information according to 401 is stored and available in the sport animal management device 10a. A server 11 is responsible for collecting all the sport animal related data for one or more sport animals from different data sources and apparatuses such as sport animal management devices 10a, different media apparatuses 12a, 12b, or different digital databases 13a, 13b, for example. The number of data sources from which the data for sport animals is collected is varying and not limited to any certain number of sources. Advantageously, the server 11 requests sport animal related data, in step 407, from the sport animal management device 10a or the sport animal management device 10a is configured to send the data to the server 11 in the end of a training event automatically without an explicit request. The sport animal management device sends back the data that is available 402. Other data sources such as different digital databases 13a or 13b and media apparatuses are also allowed to send, steps 415 and 417, their sport animal related data that they hold 414, 416 to the server 11. The server 11 receives the data sent to it 408, stores the data in step 409 based on the sport animal specific id or other identification data for sport animal as described previously above such as date of birth or name of the sport animal, for example. The purpose of storing the data with identification means is to map the data concerning a certain sport animal that has been received from different data sources and devices such as 10a, 12a, 12b, 13a, or 13b, for example, with the data that already exists in the server concerning that specific sport animal. The server 11 processes the received sport animal related data and combines it with other sport animal data that already exists in the server in order to create and maintain reference data and enable statistical analysis to be run on the data of a specific sport animal against the reference data available 410. Combining the received data with the existing reference data in the server and running statistical analysis provides analysed information on the training status and health of a specific sport animal as well as comparative information against the reference data for the specific sport animal 411. Also, the server 11 processes the new data and updates the reference data accordingly based on its own operations and receiving of new data. For example, the server 11 calculates new averages for a certain type animal group such as thoroughbred horses or 2-year-old horses.

Further in the exemplary method steps of Figure 4 a request of sport animal information for device configuration of a training event of a specific sport animal is initiated in step 403 from a sport animal management device 10b. The request advantageously contains a sport animal id for the specific sport animal or other means for identification such as date of birth, microchip id or name, stable name, or nickname of the sport animal, for example, in order to identify the animal or group of animals in question. When the server 11 receives the request in step 412, it advantageously identifies the information available on the server that relates to the specific sport animal in step 411 based on the animal id. The server 11 then creates an event or a configuration package for the requesting sport animal management device based on the analysed information available to the requested sport animal and sends that to the requesting sport animal management device 413. After receiving the information sent by the server 11 in step 404, the sport animal management device 10b advantageously configures itself based on the received information for the requested sport animal 405. After the configuration the device is ready for the training event of the specific sport animal and displays the received information about the animal 406. Alternatively, after the configuration in step 405 the device is advantageously updated with the latest information available for displaying, further analysing and studying the information, commenting or further configuring the suggested training program to be taken into use. The displayed information may contain information about the sport animal's health, previous training results and a training program for the sport animal including possible comparison to the reference data from the server, for example, as well as any feedback, notes, or instructions given by the owner or the trainer of the animal. Based on the information the sport animal management device is also advantageously capable of suggesting changes to the previous training program and instructing the trainer of the sport animal concerning possible training options.

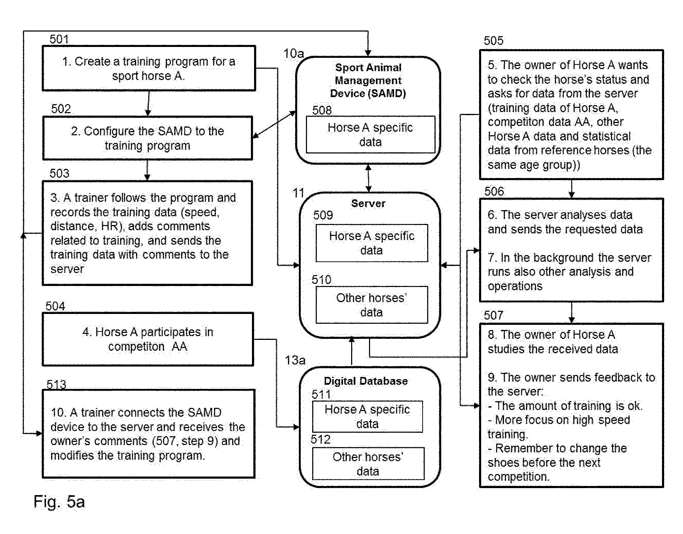

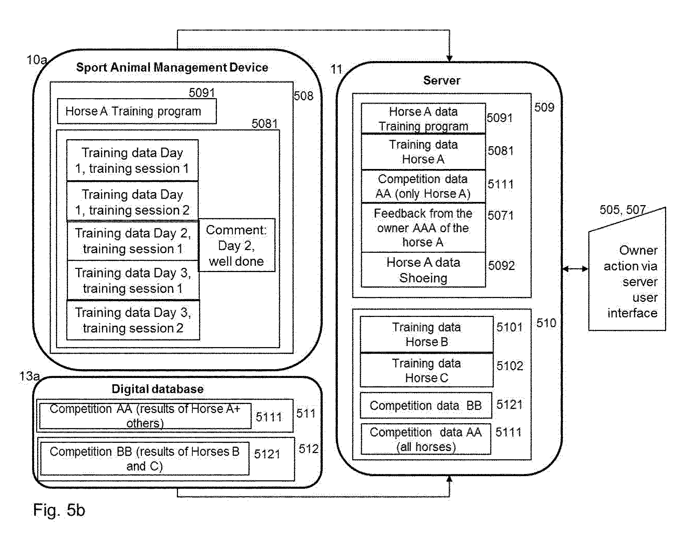

Figure 5a presents an advantageous use case based example of applying the arrangement presented in the previous exemplary embodiments in horse training and development follow-up. Figure 5b complements the use case example by presenting the arrangement of data in the exemplary use case in Figure 5a.

In the exemplary use case of Figure 5a a training program is created for a sport horse, Horse A, in the server in step 501 and saved to the data of Horse A 509. FiGure 5b shows that the training program for Horse A is stored as an item 5091 in the server 11 with the data of Horse A 509. Next in the use case according to Figure 5a, a randomly selected sport animal management device 10a will be advantageously configured with the training program of Horse A in step 502 based on the Horse A data from the server described in item 509. Figure 5b shows that the training program in the sport animal management device 5091 under the data specific to Horse A 508 matches the training program data stored in the server described in item 5091 under the data for Horse A 509. Next in the use case in Figure 5a, a trainer for Horse A follows the training program now available in the sport animal management device and training details such as speed, distance and heart rate are recorded in step 503 with sport animal management device 10a and stored in the data specific to Horse A 508. The trainer also adds a comment related to the training and the comment is saved into the data of Horse A 508. Finally, the trainer sends the training data to the server in step 503 with the sport animal management device 10a and the data is stored in the server 11 under the data for Horse A 509. Figure 5b shows that the training data of Horse A from the sport animal management device 5081 consists of training and comments on the data from five different training sessions and the training data is advantageously identified as a part of the data for Horse A also in the server described in item 5081 under the data of Horse A 509.

The next step in the use case scenario described in Figure 5a is that Horse A participates in Competition AA 504 and the results of the competition are stored in an external digital database 13a with reference to Horse A 511. Figure 5b shows that the competition results of Horse A and results of the other horses in the race are stored in the digital database 13a as an identifiable item 5111 together with the competition results BB of horses B and C 5121 and the competition data of all horses is made available at the server 11 in a manner that connects the competition results of Horse A 5111 to the data of Horse A 509 and the competition results BB 5121 and full results of competition AA 5111 to the data of other horses in the server into item 510 that is also used as a reference data in statistical analysis and sorted depending on the requested parameters for analysis.

In the use case scenario described in Figure 5a the owner of the Horse A wants to check the status of the horse and requests data of Horse A from the server in step 505. The requested data includes, for example, training data of Horse A 509 (further 5081 in Figure 5b), competition data of Horse A, other Horse A data available in the server according to item 509 (and further 5091-5092 in Figure 5b) as well as statistical comparison data from reference horses in the same age group, for example, based on the other horses' data available in the server. The server 11 is responsible for analysing the stored data and based on the analysis the server 11 sends the requested data to the owner of Horse A. The data sent to the owner includes, for example, the results of competition AA 511 (further 5111 in Figure 5b) such as placing in the competition and a chart showing the placing of Horse A during the race and the statistically analysed and compared training data such as training average for the same age group (10 hours per week, for example), Horse A's training hours (12 hours, for example) and training hours of the winner of competitions AA and BB (average 12 hours per week, for example) based on the reference data 510 (further 5101, 5102, 5121 in Figure 5b) available in the server 11 and the Horse A specific data 509. The server 11 also has shoeing data of Horse A (such as shoes were changed 30 days ago, for example) available 509 (further 5092 in Figure 5b) and this is delivered to the owner as well. In the background the server 11 runs also other analysis and operations as mentioned in the use case step 506 without an additional request from the owner or another user of the system. For example, the server 11 can process a service request such as "Horse A in Stable X in the city of XX needs shoeing in the following 2 weeks". Alternatively, the server 11 can inform or remind a stable owner or a horse manager of Horse A that the shoes should be changed. The server may compare the competition results of Horse A to the winners of the competitions and report that the difference to the winner of the latest competition has been becoming smaller over the last 12 months. The server 11 can also visualize the analysis and a chart can show that Horse A should be in par with the winning horses in two months according to the current training and performance improvement analysis.

According to the use case of Figure 5a, step 507, the owner of Horse A studies the received data and a possibility to provide feedback for Horse A is available. The owner sends a feedback for Horse A to the server 11 and the feedback is connected with the data for Horse A 509 (further 5071 in Figure 5b). The feedback can include, for example, free comments such as "the amount of training is ok, more focus on high speed training and remember to change the shoes before the next competition" 5071 (in Figure 5b). When the trainer of Horse A connects to a sport animal management device 10a and requests configuration for Horse A from the server in step 502, the trainer receives the owner's comments 5071 (in Figure 5b) through receiving the horse data stored in item 509, and the trainer can modify the training program for Horse A 5091 (in Figure 5b) based on the feedback.

Through the above presented advantageous exemplary embodiments the present invention advantageously enables an effective mechanism for collecting and combining sport animal related data and management information to a centralized system for further utilization as well as providing up-to-date, online and growing reference data pool for sport animals against which data and information related to a single sport animal can also be statistically compared. The technical effects of the invention become evident when the collected analysed information for a single sport animal available in the server (like in 211, 310 or 411, for example) is used for configuring a sport management device, 10a or 10b, for that specific sport animal for a training event with instructions based on the analysed information to the trainer of the animal for the training event (like in 205, for example). The technical effects of the invention become evident in a similar manner while configuring the sport management device 10a or 10b for presenting management information given to a specific sport animal (like in 305-306). Like the exemplary embodiments in Figures 2-4 also imply, there is no restriction that a certain sport animal would be required to use only a certain sport animal management device. On the contrary, the same sport animal management device can be used by several different sport animals or a randomly selected sport animal management device can be used for a certain animal, which is achieved through configurability as the sport animal management device is always configurable for a certain animal simply by requesting the information dedicated to that specific animal from the server (like presented in 203-206 or 303-306, for example). Also, the most up to date information is always advantageously available in the server 11. One sport animal may have more than one trainer. If one of the trainers trains the sport animal in the morning and another trainer takes a training session with the animal later during the day, they do not necessarily have the same sport animal management device 10a, 10b in use and without the configurability of the sport animal management device 10a, 10b from the server, the latest training information from the morning or notes from the morning trainer, for example, would not be available.

Further, in addition to above presented advantageous embodiments of the present invention, the effective application of the server 11 and its capabilities is achieved through a web user provided for browsing the information available in the server, running tailored analysis on the data available in the server as well as providing feedback and management information related to sport animals or adding new data for the sport animals via the web user interface 313. The web user interface to the server 11 together enables business-wise a possibility for wider utilization of the arrangement presented and allows also connecting data sources that would require manual input operations with the arrangement as well as connecting different service providers with the system. In a similar manner to the use case example of Figures 5a and 5b concerning the owner's possibility to view the information related to a certain horse or several horses, the invention can offer a possibility to share the same information with other parties as well. For example, notifications on needed shoeing, feed orders, vaccinations, or veterinarian check-ups for a horse or several horses with a certain owner or in a certain stable could be shared with wider audience such as other owners or suitable service providers in order gain maximum advantage of the possibilities that the system offers through shared orders and targeted offers, for example. This allows also the owners and stable keepers to carry our more effective budgeting and offers a possibility to save costs related to the care of their animals. For service providers it offers a possibility to expand their business effectively with a focused audience in a certain area, with certain regular needs or with certain type of animals, for example. Based on the idea of sharing the information that the invention is capable of, it can be used also in relation to betting, which is common especially in horse racing, for example. The estimations and predictions based on the actual training data, competition results, and statistically relevant and reliable comparison data would advantageously allow an effective tool in betting to estimate the possibilities and risks related to different horses, for example. Such tools are currently not available due to the same underlying problem of widely spread and decentralized data to which this invention offers a solution.

The invention being thus described, it will be obvious that the same may be varied in many ways. Such variations are not to be regarded as a departure from the scope of the invention, and all such modifications as would be obvious to one skilled in the art are intended to be included within the scope of the following claims.

## Claims

1. An arrangement for processing, collecting, analysing, and utilizing sport animal training related data, the arrangement comprising
- at least one server (11) configured to
- receive data related to at least one sport animal (208) from at least one sport animal training device (10a, 10b) or at least one media apparatus (12a, 12b) or at least one digital database (13a, 13b); and
- process and analyse the received data related to at least one sport animal (210);
- at least one sport animal management device (10a, 10b) configured to
- send and receive information request (203, 204) or management information (303, 304) for configuration of the device related to at least one sport animal to and from at least one server;
- display the information received from the server (206, 306); and
- record training event data or management information related to at least one sport animal (200, 301) and send the recorded data to at least one server (202, 302); and
- at least one media apparatus (12a,12b) or at least one digital database (13a, 13b) for providing at least competition data, veterinarian data, and service provider data related to at least one sport animal (214-217);
**characterised in that** the server (11) further comprises means for
- combining and statistically analysing the sport animal related data received from at least one sport animal management device, at least one media apparatus or at least one digital database (210) in order to provide analysed sport-animal-specific information in the server (211); and
- configuring a sport animal management device based on the analysed sport-animal-specific information received from the server (213, 204-206).

2. An arrangement according to claim 1, **characterised in that** the server (11) further comprises means for
- configuring the sport animal management device for a training event (213, 205) or management of a sport animal (312, 305) for at least one sport animal through creating a configuration data package or an event for the sport animal management device configuration (204, 304); or
- presenting the analysed information of the requested sport animal received from the server in the sport animal management device (206, 306).

3. An arrangement according to claim 2, **characterised in that** the server (11) further comprises means for
- instructing a trainer of the training program for at least one sport animal (205-206, 305-306) in the sport animal management device based on the analysed information (204, 304); or
- giving feedback or tuning a training program for at least one sport animal in the sport animal management device (205-206, 305-306) based on the analysed information (204, 304).

4. A server (11) for receiving, collecting, analysing, processing, and further sending sport animal training related data that is utilized in configuration of a sport animal management device (10a, 10b), the server (11) comprising:
- a data communication interface (111);
- one or more processors (112);
- one or more memories (113) including computer program code (1131);
- the one or more memories (113) and the computer program code (1131) configured to, with the one or more processors (112), cause the server (11) at least to
- receive with the data communication interface from at least one sport animal management device data related to at least one training event of a sport animal (208, 202);
- receive with the data communication interface from at least one media apparatus (12,a 12b) data related to at least one sport animal (208, 217); or
- receive with the data communication interface from at least one sport animal management device (10a, 10b) an information request for configuration of the said device related to at least one sport animal (212, 203);
**characterised in that** the server (11) further comprises means for sending with the data communication interface the analysed information of at least one sport animal to at least one sport animal management device in order to configure the sport animal management device (211, 213).

5. A server according to claim 4, **characterised in that** the server (11) is configured to create an event of configuring a sport animal management device or a configuration package (213) based on the analysed sport-animal-specific information (211) in order to
- configure the sport animal management device for a training event for at least one sport animal based on the analysed information in the server (213, 204-206); or
- present the analysed information of the requested sport animal in the sport animal management device (206).

6. A server according to claim 5, **characterised in that** the server (11) is configured to create an event of configuring a sport animal management device (213, 312) based on the analysed sport-animal-specific information (211, 310) in order to
- instruct a trainer of a training program for at least one sport animal based on the analysed information in the server (206); or
- give feedback or tune a training program for at least one sport animal (306).

7. A server (11) according to claim 4, **characterised in that** the server (11) is configured to receive with the data communication interface data related to a sport animal (208, 307) from at least one other digital database (13a, 13b), at least one sport animal management device (10a, 10b) or at least one media apparatus (12a 12b).

8. A server (11) according to claim 7, **characterised in that** the sport animal related data received with the data communication interface (111) from at least one other digital database (13a, 13b), at least one sport animal management device (10a, 10b) or at least one media apparatus (12a, 12b) comprises at least one of the following information: competition data, veterinarian data, service provider data, sport animal ID, birth date, gender, family tree, feeding data, shoeing data, trainer, stable keeper, training program, trainings done, training data, training index, participation of a sport animal in a certain competition, vaccination data of a sport animal, medical care of a sport animal, health condition of a sport animal, winnings of a sport animal, and results of the competitions of a sport animal.

9. A server (11) according to claim 8, **characterised in that** the received sport-animal-related data is configured to be connected with the existing data of the sport animal in the server and combined to the data of other sport animals stored in the server (210, 309).

10. A server (11) according to claim 9, **characterised in that** the received sport-animal-related data is configured to be statistically analysed and compared with the data of the sport animals stored in the server (210, 309) in order to provide analysed information of the sport animal including at least one of the following: health analysis of the sport animal, a training program for the sport animal, a development trend of the sport animal's health, and a development trend of the sport animal's performance (211,310).

11. A sport animal management device (10a, 10b) comprising
- a data communication interface (101 a, 101b);
- one or more processors (1 02a, 102b);
- one or more memories (103a, 103b) including computer program code (1031 a, 1031 b); and
- the one or more memories (103a, 103b) and the computer program code (1031 a, 1031 b) configured to, with the one or more processors (102a, 102b), cause the sport animal management device (10a, 10b) at least to
- receive with the data communication interface from at least one server information related to at least one sport animal (204, 304);
- send with the data communication interface an information request (203, 303) or management information (302) related to at least one sport animal to at least one server; and
- display the sport animal related information received from the server (203, 306);
**characterised in that** the sport animal management device further comprises means for instructing a trainer of the sport animal about a training program (205-206) or management information (305-306) for the requested sport animal based on the analysed information received from the server (204, 304).

12. A sport animal management device (10a, 10b) according to claim 11, **characterised in that** the sport animal management device is configured to record training event data for at least one sport animal (200).

13. A sport animal management device (10a, 10b) according to claim 12, **characterised in that** the sport animal management device is configured to send recorded training event data from at least one training event of a training sport animal to a server for storing and further processing of the data (202).

14. A method for processing, collecting, analysing, and utilizing sport animal training related data, the method comprising
- receiving from at least one sport animal management device (10a, 10b) data related to at least one training event or management information of a sport animal in the server (408);
- receiving from at least one media apparatus (12a, 12b) or digital database (13a, 13b) data related to at least one sport animal in the server (408);
- sending an information request related to at least one sport animal from at least one sport animal management device to at least one server (403) and receiving a response to the request in the sport animal management device (404); and
- receiving from at least one sport animal management device an information request related to at least one sport animal in the server (412) and responding to the request in the server (413);
**characterised in that** the method further comprises
- combining and statistically analysing the sport animal related data received from at least one sport animal management device, at least one media apparatus or at least one digital database in the server (409, 410) in order to provide analysed sport-animal-specific information (411); and
- configuring a sport animal management device based the analysed sport-animal-specific information received from the server (405, 406).

15. A method according to claim 14, **characterised in that** the method further comprises
- configuring the sport animal management device for a training event or management of a sport animal for at least one sport animal based on the analysed information through creating an event of configuration or a package for configuration of the said device (405);
- instructing a trainer of the training program for at least one sport animal in the sport animal management device based on the analysed information received from the server (406);
- giving feedback or tuning a training program for at least one sport animal in the sport animal management device based on the analysed information received from the server (406); or
- presenting the analysed information of the requested sport animal received from the server in the sport animal management device (406).
